# EUROPEAN PATENT APPLICATION

(11) **EP 4 734 119 A1**
(43) Date of publication of application: **29.04.2026**
(21) Application number: 25210671.1
(22) Date of filing: 23.10.2025
(51) Int. Cl.: G16C 20/50, G16C 20/70

(54) **REINFORCEMENT LEARNING PROGRAM, METHOD FOR REINFORCEMENT LEARNING, AND INFORMATION PROCESSING APPARATUS**

(30) Priority: 25.10.2024 JP 2024188327
(71) Applicant: FUJITSU LIMITED, Kawasaki-shi, Kanagawa 211-8588 (JP)
(72) Inventor: KOMIKADO, Tao, Kawasaki-shi, Kanagawa, 211-8588 (JP)
(74) Representative: Hoffmann Eitle

(57) **Abstract**

A reinforcement learning program that causes a computer to execute a process including: executing reinforcement learning of a machine learning model (21a), using a first three-dimensional (3D) molecular structure of a first compound to be adapted to a first object as a state, assignment of a type of an additional atom, a binding position of the additional atom to the first 3D molecular structure, and a 3D position of the additional atom, to the first compound in the state as an action, and an index to evaluate a second 3D molecular structure obtained by binding the additional atom to the first 3D molecular structure in the action as a reward.

## Description

### [Technical Field]

The embodiment discussed herein relates to a reinforcement learning program, a method for reinforcement learning, and an information processing apparatus.

### [Background Art]

In the field of Artificial Intelligence (AI) drug discovery, De Novo Drug Design by means of generative models has been studied.

Examples of an index for evaluating a compound produced by means of a generative model include indexes using chemical property, such as Quantitative Estimate of Drug-likeness (QED) and a Synthetic Accessibility Score (SA Score).

These indexes are not differentiatable and therefore are not allowed to be optimized in the scheme of normal supervised machine learning. As a solution to the above, a method for optimizing QED and an SA score by means of reinforcement learning has been known.

### [Prior Art Reference]

### [Non-Patent Document]

[Non-Patent Document 1] "Graph Convolutional Policy Network for Goal-Directed Molecular Graph Generation", Jiaxuan You et al.,arXiv:1806.02473v3 [cs.LG] 25 Feb 2019, <Internet: arxiv.org/abs/1806.02473>, [searched on March 11, 2024]
[Non-Patent Document 2] "GraphAF: a Flow-based Autoregressive Model for Molecular Graph Generation", Chence Shi et al.,arXiv:2001.09382v2 [cs.LG] 27 Feb 2020, <Internet: arxiv.org/abs/2001.09382>, [searched on March 11, 2024]
[Non-Patent Document 3] "Hit and Lead Discovery with Explorative RL and Fragment-based Molecule Generation", Soojung Yang et al.,35th Conference on Neural Information Processing Systems (NeurIPS 2021), <Internet: proceedings.neurips.cc/paper_files/paper/2021/file/41da609c5 19d77b29be442f8c1105647-Paper.pdf>, [searched on March 19, 2024]
[Non-Patent Document 4] "Molecule Generation For Target Protein Binding with Structural Motifs", Zaixi Zhang, Published as a conference paper at ICLR 2023, <Internet: openreview.net/forum?id=Rq13idF0F73>, [searched on March 11, 2024]

### [Summary of Invention]

### [Problems to be Solved by Invention]

Unfortunately, the above method, which treats a two-dimensional (2D) compound graph expression, may have difficulty in specifying a three-dimensional (3D) molecular structure of an appropriate compound for an object. For example, since the above method does not take advantage of the 3D protein pocket structure of the object and the 3D compound graph expression of a compound adapted to the object, the above method may have difficulty in generating a compound graph that is well adapted three-dimensionally to the protein pocket.

The case where it is difficult to specify a 3D molecular structure of the appropriate compound for the object is not limited to the above-described case of new drug development of AI drug discovery, and is similarly applied to a case where various 3D molecular structures are specified for the purpose of, for example, material development.

Accordingly, it is an object in one aspect of the embodiment to make it possible to specify a 3D molecular structure of an appropriate compound for an object.

### [Means to Solve the Problem]

According to an aspect of the embodiment, a reinforcement learning program that causes a computer to execute a process including: executing reinforcement learning of a machine learning model, using a first three-dimensional (3D) molecular structure of a first compound to be adapted to a first object as a state, assignment of a type of an additional atom, a binding position of the additional atom to the first 3D molecular structure, and a 3D position of the additional atom, to the first compound in the state as an action, and an index to evaluate a second 3D molecular structure obtained by binding the additional atom to the first 3D molecular structure in the action as a reward.

### [Effect of Invention]

According to an aspect of the one embodiment, a 3D molecular structure of an appropriate compound for an object can be specified.

### [Brief Description of Drawings]

FIG. 1 is a block diagram illustrating an example of a configuration of a reinforcement learning unit according to one embodiment;
FIG. 2 is a block diagram illustrating an example of a hardware configuration of a computer that achieves the function of a server according to an example of the one embodiment;
FIG. 3 is a block diagram illustrating an example of a software configuration of the server according to an example of the one embodiment;
FIG. 4 is a diagram illustrating an example of a machine learning process of a machine learning model;
FIG. 5 is a diagram illustrating an example of a reinforcement learning process of the machine learning model;
FIG. 6 is a flow diagram illustrating an example of operation of the reinforcement learning process of the machine learning model in the server according to the one embodiment;
FIG. 7 is a flow diagram illustrating an example of operation of the machine learning process of the machine learning model in the server according to the one embodiment; and
FIG. 8 is a flow diagram illustrating an example of operation of a machine inference process of the machine learning model in the server according to the one embodiment.

### [Description of Embodiments]

Hereinafter, an embodiment will now be described with reference to the accompanying drawings. However, the following embodiment is merely illustrative and is not intended to exclude the application of various modifications and techniques not explicitly described in the embodiment. For example, the present embodiment can be variously modified and implemented without departing from the scope thereof. Further, each of the drawings can include additional functions not illustrated therein to the elements illustrated in the drawings.

### (A) Description of Reinforcement Learning Unit of One Embodiment

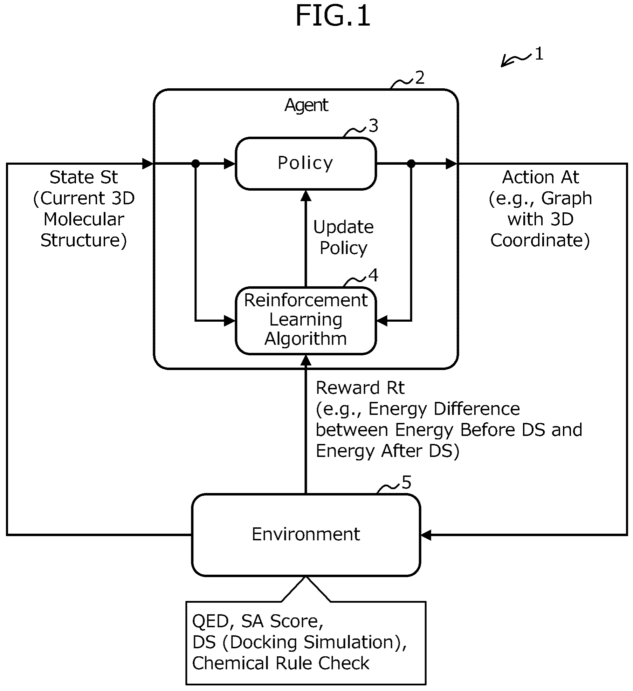

FIG. 1 is a block diagram illustrating an example of a configuration of a reinforcement learning unit 1 according to one embodiment. The reinforcement learning unit 1 executes reinforcement learning (reinforcement training) of a machine learning model such that a 3D molecular structure of an appropriate compound for an object can be specified.

In the following description, a 3D molecular structure is exemplified by a 3D compound graph (which may be referred to as a 3D molecular graph). In the one embodiment, an example of an object (first object) is a pocket structure of a protein, and an example of a compound appropriate for the object is a compound (which may be referred to as a generated compound) well adapted three-dimensionally to the pocket of the protein. The 3D molecular structure, the object, and the compound according to the one embodiment are not limited thereto.

The reinforcement learning unit 1 may include an agent 2 and an environment 5. The agent 2 selects an action At from among an action space As_t including possible actions that the agent 2 may take in the environment 5 and outputs the selected action At to the environment 5. The environment 5 observes and evaluates the action At, and outputs a state St that results from the observed action At and a reward Rt deserving the evaluation result to the agent 2. The reward Rt may include, for example, an index that evaluates an appropriate (good) action At.

The agent 2 may include a policy 3 and a reinforcement learning algorithm 4. The policy 3 generates and outputs the action At in response to an input of a current state St therein. An example of the policy 3 is a machine learning model. The reinforcement learning algorithm 4 updates the policy 3 on the basis of the current state St, the action At, and the reward Rt. As the reinforcement learning algorithm 4, various algorithms such as Proximal Policy Optimization (PPO) may be used.

As the above, the agent 2 repeats the process of generating a new action At from the state St that results from the action At and updating the policy 3 in response to a new state St and a new reward Rt related to the new action At. This makes it possible to refine (fine-tune) the policy 3 to take an appropriate action At that maximizes the reward Rt.

Here, the above-described method for optimizing QED and an SA score by reinforcement learning treats a 2D compound graph expression. For this reason, the method may have difficulty in generating a compound graph that is well adapted three-dimensionally to a protein pocket.

In contrast to the above, the reinforcement learning unit 1 according to the one embodiment can specify a 3D molecular structure of an appropriate compound for an object by executing reinforcement learning of a machine learning model, for example, the policy 3, using a state St, an action At, a reward Rt to be described below.

The reinforcement learning unit 1 may use, as the state St, a 3D molecular structure (first 3D molecular structure) of a first compound to be adapted to a first object. For example, the state St may be a 3D molecular structure obtained by binding an additional atom to the 3D molecular structure in the previous action At.

The reinforcement learning unit 1 may use, as an action At, assignment of the type of an additional atom to the first compound in the state St, assignment of a binding position of the additional atom to the 3D molecular structure to the first compound in the state St, and assignment of a 3D position of the additional atom to the first compound in the state St. The 3D position of the additional atom may be, for example, information about the 3D coordinate of the additional atom.

The reinforcement learning unit 1 may use, as a reward Rt, an index for evaluating a 3D molecular structure (second 3D molecular structure) obtained by biding the additional atom to the 3D molecular structure in the action At.

With this configuration, the reinforcement learning unit 1 executes reinforcement learning of the policy 3 such that the reward Rt related to an action At including assignment of the position on the 3D coordinates of the additional atom in addition to assignment of the type and the biding position of the additional atom is maximized. As described above, since the policy 3 is refined by the reinforcement learning such that the 3D molecular structure obtained by the action At comes to be better, the reinforcement learning unit 1 can specify the 3D molecular structure of an appropriate compound for the first object. Refining of the policy 3 may include, for example, updating of a parameter of a machine learning model serving as a model of the policy 3.

The index of the reward Rt may include a first index related to the affinity of binding (docking) between a 3D structure of the first object and the 3D molecular structure obtained in the action At. An example of the affinity of biding (docking) is a result of Docking Simulation (DS) of the first object and the first compound. Various known techniques may be used as the DS.

By using the first index as the index of the reward Rt, it is possible to specify a 3D molecular structure of a (appropriate) compound having higher affinity for the first object, in other words, having better three-dimensional fittability. The 3D molecular structure of an appropriate compound may be referred to as, for example, the 3D molecular structure of a feasible or practical compound.

In addition, the first index may include an energy difference between a total energy and an entire energy. The total energy is a sum of an energy of the 3D structure of the first object and an energy of the 3D molecular structure obtained in the action At. The entire energy is an energy when a position of binding between the 3D structure of the first object and the 3D molecular structure obtained in the action At is optimum.

An example of the entire energy is the energy when the arrangement (conformation) of the first object and the first compound obtained by the DS is optimized, which means the energy after the DS or an energy of a composite of the first object and the first compound obtained in the action At. An example of the total energy is the sum of an energy of the 3D structure of the first object before the DS and an energy of the 3D molecular structure obtained by the action At before the DS, in other words, the sum of the energies before the DS.

Since a larger energy difference means that the first compound has better 3D fittability to the protein pocket, it is possible to specify the 3D molecular structure of a (appropriate) compound having better 3D fittability to the first object.

The indexes of the reward Rt may further include at least one of a second index and a third index. The second index is an index relating to a likelihood that the 3D molecular structure obtained in the action At is medicine, and is exemplified by QED. The third index is an index relating to a difficulty in binding between the 3D structure of the first object and the 3D molecular structure obtained in the action At, and is exemplified by an SA score.

By further using at least one of the second index and the third index in addition to the first index, it is possible to specify the 3D molecular structure of a more appropriate compound for the first object.

The reinforcement learning algorithm 4 may execute the reinforcement learning of the policy 3 such that the reward Rt is maximized while giving a higher priority to the first index than the second and/or third indexes among the multiple indexes included in the reward Rt.

In calculating the above indexes, or in addition to calculating the above indexes, the environment 5 of the reinforcement learning unit 1 may carry out chemical rule check on the generated compound and apply its result to the indexes.

The agent 2 of the reinforcement learning unit 1 may use, as a model of the policy 3, a trained machine learning model after being trained so as to output a 3D molecular structure of a second compound to be adapted to each of multiple second objects to be input, including the first object, in response to input of the second object into the machine learning model. On an assumption that the first object is a protein or its pocket structure, the multiple second objects are exemplified by various proteins including the protein.

In other words, it can be said that the trained machine learning model is trained to be a generic feature extractor for various proteins. For example, since a disease-related protein exists in a particular disease, an appropriate compound for the first object is to be generated in actual working for drug discovery, assuming the protein to be the first object.

As an example, the reinforcement learning by the reinforcement learning unit 1 may be performed after narrowing a protein and which pocket structure of the protein that a drug discovery researcher is interested in. The reinforcement learning unit 1 may refine (fine-tune) the generic feature extractor to fit into a narrowed particular pocket structure of a narrowed particular protein by means of reinforcement learning executed with a fixed pocket structure. This makes it possible to generate a compound generator (model of the policy 3 completing the reinforcement learning) that optimizes the 3D fittability in addition to the chemical property (characteristic) such as QED and an SA score.

As described above, since the use of a machine learning model after being trained as a generic feature extractor as a model of the policy 3 also matches the scheme in the field of the actual working for drug discovery, it is possible to specify the 3D molecular structure of a more feasible or practical compound, for example.

The base of the model of the policy 3 is exemplified by a Fragment based LigAnd Generation framework (FLAG) model that executes fragment-based Structure Based Drug Design (SBDD) in a 3D graph.

The reinforcement learning unit 1 may use, as an example of the model of the policy 3, a trained FLAG model (hereinafter, simply referred to as "FLAG") after being trained to be a generic feature extractor for various proteins. In the following description, the model of the policy 3 is assumed to be a trained FLAG model, but is not limited thereto. Alternatively, various trained machine learning models may be used as the model of the policy 3.

In addition, the model of the policy 3 is not limited to a fragment-based model, and may alternatively be an atom-based model.

The FLAG links fragments in an auto regressive manner. For the above, the action space As_t of the FLAG may be configured by selecting one or more nodes accompanying 3D coordinates on each graph from among a group of possible novel structures obtained while a new fragment is predicted.

Here, the FLAG generates (outputs), when a pocket structure is given, a three-dimensional compound graph fittable to the given pocket structure, but does not generate a compound considering QED, an SA score and/or an energy difference between before and after the DS.

For the above, the reinforcement learning unit 1 executes the reinforcement learning of the FLAG such that the FLAG outputs, as the action At, a compound (fragment) that is fitted to the given pocket structure and that brings good results with indexes such as the QED, the SA score, and the energy difference between the energies before and after the DS.

### (B) Example of Configuration of Server of One Embodiment

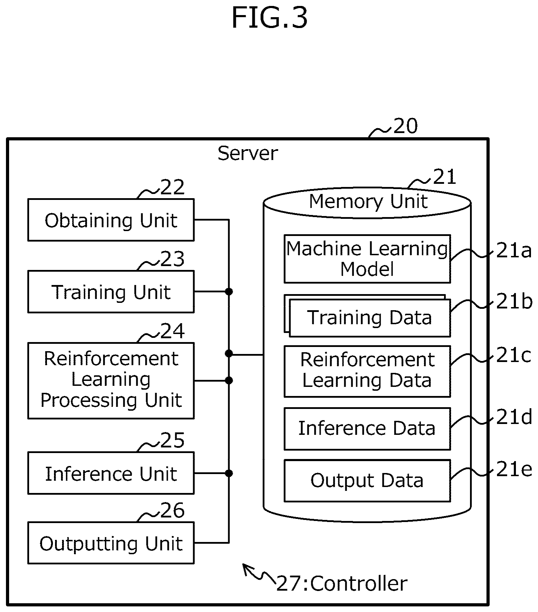

Description will now be made in relation to an example of a configuration of a server 20 (see FIG. 3) achieving the reinforcement learning unit 1 of the one embodiment described above.

### (B-1) Hardware Configuration:

The server 20 of the one embodiment may be a virtual server (VM: Virtual Machine) or a physical server. The function of the server 20 may be achieved by one computer or by two or more computers. Further, at least a part of the function of the server 20 may be implemented using Hardware (HW) resources and Network (NW) resources provided by cloud environment.

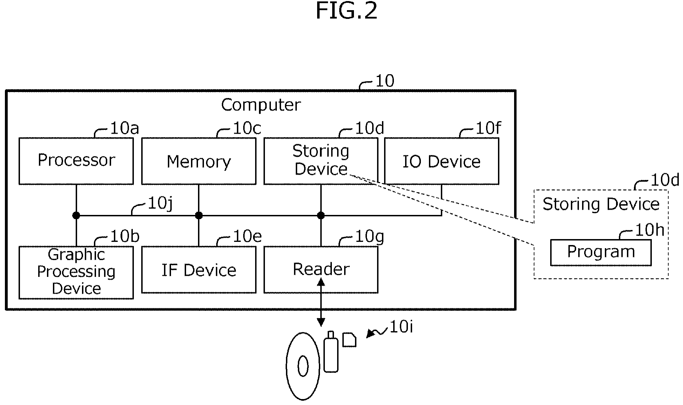

FIG. 2 is a block diagram schematically illustrating an example of a hardware (HW) configuration of a computer 10 that achieves the function of the server 20 according to an example of the one embodiment. If multiple computers are used as the HW resources for achieving the function of the server 20, each of the computers may include the HW configuration illustrated in FIG. 2.

As illustrated in FIG. 2, the computer 100 may illustratively include, as the HW configuration, a processor 10a, a graphic processing device 10b, a memory 10c, a storing device 10d, an Interface (IF) device 10e, an Input/Output (IO) device 10f, and a reader 10g.

The processor 10a is an example of an arithmetic processing device that performs various types of control and calculation. The processor 10a may be communicably connected to each of the blocks in the computer 10 via a bus 10j. The processor 10a may be a multi-processor including multiple processors or a multi-core processor including multiple processor cores, or may have a structure including two or more multi-core processors.

The processor 10a may be any one of integrated circuits (ICs) such as Central Processing Units (CPUs), Micro Processing Units (MPUs), Accelerated Processing Units (APUs), Digital Signal Processors (DSPs), Application Specific Integrated Circuits (ASICs), and Field Programmable Gate Arrays (FPGAs), or combinations of two or more of these ICs.

The graphic processing device 10b controls screen-displaying on an output device such as a monitor display among the IO device 10f. Further, the graphic processing device 10b may have a configuration serving as an accelerator that executes a machine learning process, a reinforcement learning process, and an inference process using a machine learning model. Examples of the graphic processing device 10b are various ICs such as Graphic Processing Units (GPUs), APUs, DSPs, ASICs, and FPGAs.

The memory 10c and the storing device 10d each store information such as various pieces of data and programs. An example of the memory 10c is one of a volatile memory such as a Dynamic Random Access Memory (DRAM) and a non-volatile memory such as a Persistent Memory (PM) or the both. Examples of the storing device 10d may be various storing devices including a magnetic disk device such as a Hard Disk Drive (HDD), a semiconductor drive device such as a Solid State Drive (SSD), a nonvolatile memory, and the like. The non-volatile memory may be, for example, a flash memory, a Storage Class Memory (SCM), a Read Only Memory (ROM), and the like.

The storing device 10d may store a program 10h (reinforcement learning program) that implements all or a part of various functions of the computer 10. For example, the processor 10a of the server 20 may achieve the function of a controller 27 to be detailed below (see FIG. 3) by expanding the program 10h stored in the storing device 10d on the memory 10c and executing the expanded program 10h.

The IF device 10e is an example of a communication IF that controls the connection and communication between the server 20 and another computer. For example, the IF device 10e may include an adapter conforming to electrical communication (e.g., Local Area Network (LAN)) such as Ethernet^{®} or optical communication such as Fibre Channel (FC). The adapter may be compatible with either one or each of wireless and wired communication schemes. Furthermore, the program 10h may be downloaded from a network to the computer 10 through the communication IF and be stored in the storing device 10d.

The IO device 10f may include one or each of an input device and an output device. Examples of the input device include a keyboard, a mouse, and a touchpanel. Examples of the output device include a monitor, a projector, and a printer. The IO device 10f may include, for example, a touchpanel that integrates an input device and a displaying device with each other. The output device may be connected to the graphic processing device 10b.

The reader 10g is an example of a reader that reads information on data and programs recorded in a recording medium 10i. The reader 10g may include a connecting terminal or device to which the recording medium 10i can be connected or inserted. Examples of the reader 10g include an adapter conforming to, for example, a Universal Serial Bus (USB), a drive apparatus that accesses a recording disk, and a card reader that accesses a flash memory such as an SD card. The program 10h may be stored in the recording medium 10i. The reader 10g may read the program 10h from the recording medium 10i and store the read program 10h into the storing device 10d.

Examples of the recording medium 10i illustratively include a non-transitory computer-readable recording medium such as a magnetic/optical disk, and a flash memory. Examples of the magnetic/optical disk illustratively include a flexible disk, a Compact Disc (CD), a Digital Versatile Disc (DVD), a Blu-ray disk, and a Holographic Versatile Disc (HVD). An example of the flash memory illustratively includes a semiconductor memory such as a USB memory and an SD card.

The HW configuration of the computer 10 described above is exemplary. Accordingly, the computer 10 may appropriately undergo increase or decrease of the HW devices (e.g., addition or deletion of arbitrary blocks), division, integration in an arbitrary combination, or addition or deletion of the bus.

### (B-2) Example of Software Configuration

FIG. 3 is a block diagram illustrating an example of a software configuration of the server 20 according to an example of the one embodiment. The server 20 is an example of a computer or an information processing apparatus that executes reinforcement learning (reinforcement training) of a machine learning model.

As illustrated in FIG. 3, the server 20 may illustratively include a memory unit 21, an obtaining unit 22, a reinforcement learning processing unit 24, and an outputting unit 26. In addition, the server 20 may further include a training unit 23 and an inference unit 25. The obtaining unit 22, the training unit 23, the reinforcement learning processing unit 24, the inference unit 25, and the outputting unit 26 are an example of the controller 27. The reinforcement learning processing unit 24 is an example of the reinforcement learning unit 1 described with reference to FIG. 1.

The memory unit 21 is an example of a storing region, and stores various types of data that the server 20 uses. The memory unit 21 may be implemented by, for example, a storing region included in one or each of the memory 10c and the storing device 10d (see FIG. 2) of the server 20.

As illustrated in FIG. 3, the memory unit 21 may illustratively be capable of storing a machine learning model 21a and reinforcement learning data 21c. In addition, if the server 20 includes the training unit 23, the memory unit 21 may be capable of storing multiple pieces of training data 21b. Further, if the server 20 includes the inference unit 25, the memory unit 21 may be capable of storing inference data 21d and output data 21e. The memory unit 21 may store these data in various format such as a Database (DB), a file, or an array.

The machine learning model 21a is an example of the model of the policy 3, and may be, for example, a FLAG model.

The multiple pieces of the training data 21b are data to be used in a machine learning process (training) of the machine learning model 21a. The multiple pieces of the training data 21b may be referred to as a training data set. The training data 21b may include, for example, multiple combinations (sets) of a 3D structure of a pocket structure of a protein serving as an example of a second object and a 3D molecular structure of a second compound appropriate for the second object. The multiple combinations are provided one for each of a protein type or a pocket structure type.

The reinforcement learning data 21c is data to be used in a reinforcement learning process on a trained machine learning model 21a. An example of the reinforcement learning data 21c includes a 3D structure of a particular pocket structure of a particular protein serving as an example of the first object.

The inference data 21d is data to be used in an inference process on the machine learning model 21a after being subjected to training and reinforcement learning. An example of the inference data 21d is the three-dimensional structure of a certain pocket structure of a certain protein that is an example of an inference object. The inference object may be the same as or related to the first object.

The output data 21e is data to be output from the machine learning model 21a in the reinforcement learning process or the inference process, and may include, for example, information on the action At.

The obtaining unit 22 obtains various types of information to be used in the server 20. For example, the obtaining unit 22 may obtain at least one type of data among the machine learning model 21a, the training data 21b, the reinforcement learning data 21c, and the inference data 21d from one or more devices (not illustrated) that provide various types of information, and may store the obtained data in the memory unit 21. The machine learning model 21a may have completed training or may be a neural network (NN) that has not been trained or has not completed training yet.

The training unit 23 executes a machine learning process (supervised machine learning processing, training) of the machine learning model 21a using the multiple pieces of the training data 21b in a training phase.

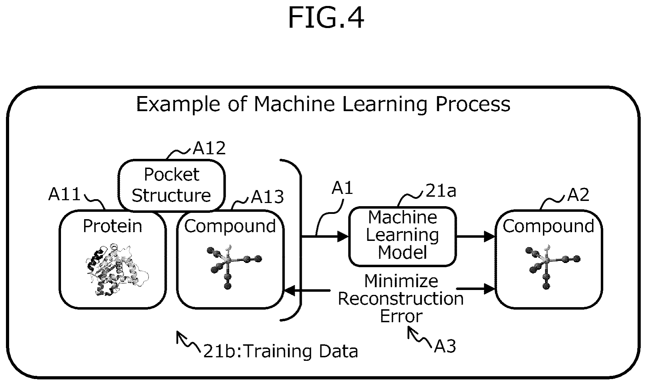

FIG. 4 is a diagram illustrating an example of the machine learning process of the machine learning model 21a (e.g., a FLAG model).

As illustrated in FIG. 4, the training data 21b may include a combination of an object (second object) containing a protein A11 and its pocket structure A12 and an appropriate compound A13 (correct answer data, ground truth) with respect to the pocket structure A12.

For example, the training unit 23 inputs the protein A11 and its pocket-structured A12 contained in the training data 21b into the machine learning model 21a (see Arrow A1).

The training unit 23 obtains the compound A2 output from the machine learning model 21a in response to the input of the training data 21b. The training unit 23 updates (optimizes) a parameter of the machine learning model 21a such that the obtained compound A2 and the compound A13 contained in the training data 21b match with each other (which means that the error is minimized), in other words, such that the compound A2 can be reconstructed (see Arrow A3).

The training unit 23 trains the machine learning model 21a by executing the above process on each piece of the training data 21b. As a method for determining the end of the machine learning process of the machine learning model 21a, various known methods may be adopted.

If the obtaining unit 22 obtains the trained machine learning model 21a from another device, the process and the configuration of the training unit 23 may be omitted.

In the training phase, the reinforcement learning processing unit 24 executes the reinforcement learning process of the trained machine learning model 21a, using the reinforcement learning data 21c. For example, the reinforcement learning processing unit 24 can execute the process as the reinforcement learning unit 1 according to the one embodiment described with reference to FIG. 1.

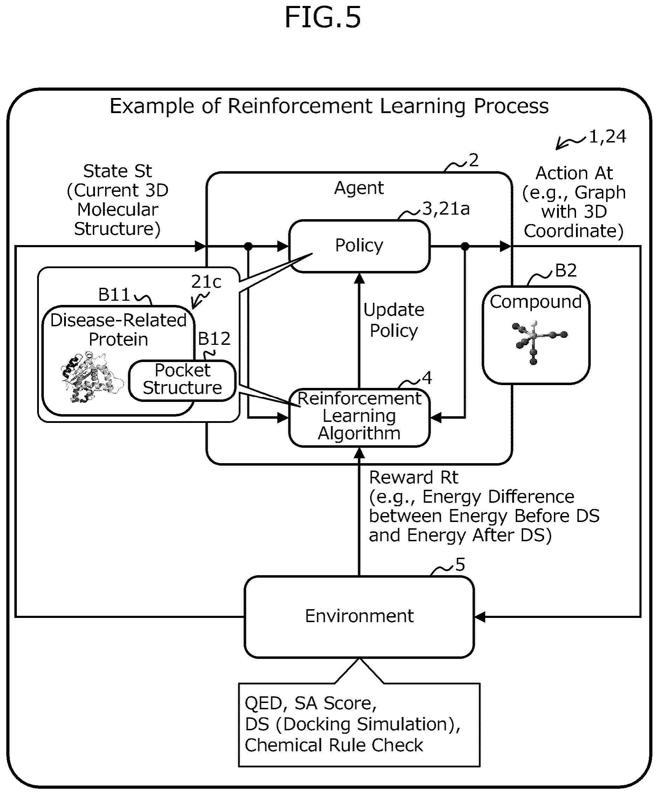

FIG. 5 is a diagram illustrating an example of the reinforcement learning process of the machine learning model 21a (e.g., FLAG model).

As illustrated in FIG. 5, the reinforcement learning data 21c may include an object (first object) including a particular protein B11 and its pocket structure B12.

For example, the reinforcement learning processing unit 24 may be set to fixedly input the particular protein B11 and its pocket structure B12 contained in the reinforcement learning data 21c into the machine learning model 21a (i.e., the policy 3). From the machine learning model 21a, a compound B2 which is one example of the action At is output.

The reinforcement learning processing unit 24 may, via the reinforcement learning algorithm 4, update the parameter of the machine learning model 21a on the basis of a compound serving as an example of a current state St and the compound B2 serving as an example of an action At such that the reward Rt including an energy difference between before and after the DS increases. In other words, the reinforcement learning processing unit 24 may perform training (fine tuning) of the machine learning model 21a. The reinforcement learning processing unit 24 may use the particular protein B11 and its pocket structure B12 in the process of the reinforcement learning algorithm 4.

The reinforcement learning processing unit 24 may execute a DS that binds the compound B2 serving as an example of an action At to the particular protein B11 and its pocket structure B12 in the environment 5. The reinforcement learning processing unit 24 may include, in the reward Rt, an energy difference between a total energy and an entire energy and output the energy difference to the reinforcement learning algorithm 4. The total energy is a sum of the energy of the particular protein B11 and its pocket structure B12 and the energy of the compound B2. The entire energy is an energy obtained by the DS, which means the energy of a composition obtained by the DS. The energy differences may be, for example, a value calculated by subtracting the entire energy from the total energy.

In addition, the reinforcement learning processing unit 24 may execute at least one of the QED and the SA score on the compound B2 in the environment 5, and may include the result of the execution into the reward Rt.

As a method for determining the end of the reinforcement learning process of the machine learning model 21a, various known methods may be adopted. The reinforcement learning processing unit 24 may store, as the output data 21e, the 3D molecular structure of the compound B2 finally obtained by the reinforcement learning process into the memory unit 21.

In an inference phase, the inference unit 25 executes the inference process using the machine learning model 21a that has been trained (reinforcement-learned) by the reinforcement learning processing unit 24. For example, the inference unit 25 may input a protein and its pocket structure contained in the inference data 21d into the machine learning model 21a (i.e., policy 3). Then, the inference unit 25 may obtain the 3D molecular structure of the compound serving an example of an action At output from the machine learning model 21a. The inference unit 25 may store the obtained 3D molecular structure of the compound as the output data 21e into the memory unit 21.

The outputting unit 26 outputs data. The data to be output includes, for example, at least one type of data among the machine learning model 21a after being subjected to the training and the reinforcement learning and the output data 21e. The data to be output may include various intermediate data generated during the process executed by the training unit 23, the reinforcement learning processing unit 24, or the inference unit 25.

In outputting the data, the outputting unit 26 may transmit (provide) the data to non-illustrated another computer, or may accumulate the data into the memory unit 21 to manage the data to be obtainable from the server 20 or another computer. Alternatively, the outputting unit 26 may screen-output the data or information representing the data onto, for example, an output device of the server 20 or an administrator terminal, or may output the data in various other manners. The administrator terminal is an example of a computer used by an administrator.

### (C) Example of Operation

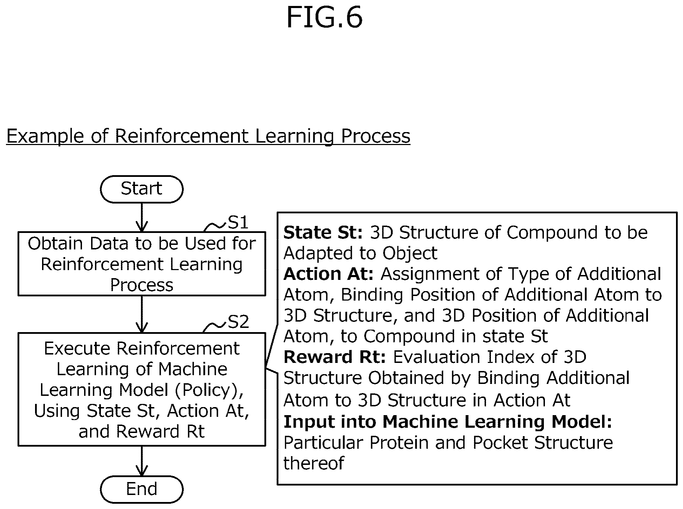

Next, description will now be made in relation to an example of the operation in the server 20 serving as an example of the one embodiment configured as the above with reference to FIGS. 6 to 8.

### (C-1) Example of Reinforcement Learning Process

FIG. 6 is a flow diagram illustrating an example of operation of the reinforcement learning process of the machine learning model 21a in the server 20 according to the one embodiment.

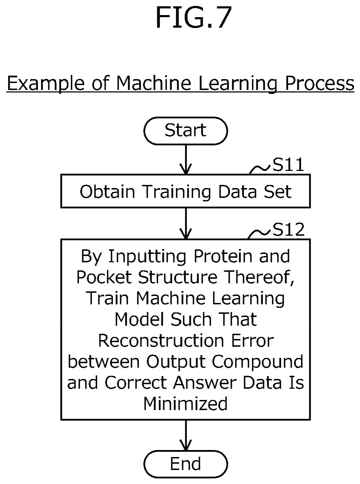

As illustrated in FIG. 6, the obtaining unit 22 obtains data to be used for the reinforcement learning process, for example, the trained machine learning model 21a and the reinforcement learning data 21c (Step S1). The server 20 may execute a machine learning process to be described below with reference to FIG. 7 by obtaining a training data set (i.e., the multiple pieces of the training data 21b) in place of the trained machine learning model 21a obtained by the obtaining unit 22.

The reinforcement learning processing unit 24 executes the reinforcement learning of the machine learning model 21a (policy 3) using the state St, the action At, and the reward Rt (Step S2), and ends the process if a finish condition of the reinforcement learning is satisfied.

In the reinforcement learning, the state St, the action At, and the reward Rt are set as follows, for example. The state St is a 3D structure of the compound to be adapted to the object. The action At is assignment of a type of an additional atom, a biding position of the additional atom to the 3D structure, and a 3D position of the additional atom, to the compound in the state St. The reward Rt is an evaluation index of a 3D structure obtained by binding the additional atom to the 3D structure in the action At.

Also, in the reinforcement learning, a particular protein and its pocket structure contained in the reinforcement learning data 21c are fixed as the input to the machine learning model 21a.

The outputting unit 26 may output information related to the machine learning model 21a after subjected to reinforcement learning, information related to a compound finally obtained in the reinforcement learning process, and intermediate data in the reinforcement learning process, for example.

### (C-2) Example of Machine Learning Process

FIG. 7 is a flow diagram illustrating an example of operation of the machine learning process of the machine learning model 21a in the server 20 according to the one embodiment. The training unit 23 may execute, instead of or in addition to obtaining of the trained machine learning model 21a by the obtaining unit 22, training of the machine learning model 21a.

As illustrated in FIG. 7, the obtaining unit 22 obtains the training data set (multiple pieces of the training data 21b) (Step S11).

The training unit 23 inputs the protein and its pocket structure contained in each of the multiple pieces of the training data 21b into the machine learning model 21a. The training unit 23 trains the machine learning model 21a such that the reconstruction error between the compound output from the machine learning model 21a and the correct answer data in the training data 21b is minimized (Step S12), and ends the process if a finish condition of the machine learning is satisfied.

The outputting unit 26 may output information related to the trained machine learning model 21a, and intermediate data in the machine learning process, for example.

### (C-3) Example of Inference Process

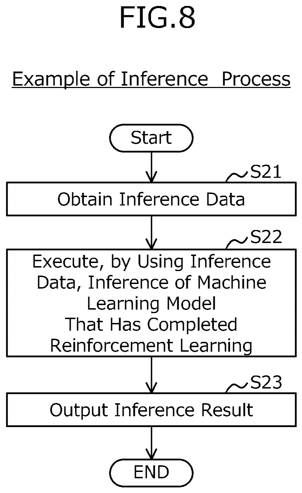

FIG. 8 is a flow diagram illustrating an example of operation of the inference process of the machine learning model 21a by the server 20 according to the one embodiment.

As illustrated in FIG. 8, the obtaining unit 22 obtains the inference data 21d (Step S21).

The inference unit 25 executes the inference process by using the inference data 21d, for example, using a protein and its pocket structure contained in the inference data 21d as an input into the machine learning model 21a (Step S22).

The inference unit 25 obtains a result (inference result) of the inference process from the machine learning model 21a, which result is exemplified by information on the 3D molecular structure of the compound.

The outputting unit 26 outputs the inference result (Step S23), and the inference process ends. For example, the outputting unit 26 may store (output) the inference result as the output data 21e into the memory unit 21. In addition, the outputting unit 26 may also output intermediate data in the inference process, for example.

### (D) Miscellaneous

The technique according to the one embodiment described above can be implemented by changing or modifying as follows.

For example, the elements 21 to 26 included in the server 20 illustrated in FIG. 3 may be merged in any combination or may each be divided.

Further, for example, the server 20 illustrated in FIG. 3 may have a configuration in which multiple apparatuses cooperate with each other via a network to implement respective process functions. As an example, the obtaining unit 22, the training unit 23, the reinforcement learning processing unit 24, the inference unit 25, and the outputting unit 26 may be implemented by an application server or a web server, and the memory unit 21 may be implemented by a DB server. In this case, the process functions as the server 20 may be achieved by the web server, the application server, and the DB server cooperating with one another via a network.

Furthermore, the described one embodiment assumes a method for specifying a 3D molecular structure of an appropriate compound for a first object in the field of, for example, new drug development such as AI drug discovery, but the application of the one embodiment is not limited to this. The method of the one embodiment can be similarly applied to a case where 3D molecular structures of appropriate compounds for various first objects are specified for the purpose of material development, for example.

### [Reference Signs List]

- 1 :: reinforcement learning unit
- 2 :: agent
- 3 :: policy
- 4 :: reinforcement learning algorithm
- 5 :: environment
- 10 :: computer
- 20 :: server
- 21 :: memory unit
- 21a :: machine learning model
- 21b :: training data
- 21c :: reinforcement learning data
- 21d :: inference data
- 21e :: output data
- 22 :: obtaining unit
- 23 :: training unit
- 24 :: reinforcement learning processing unit
- 25 :: inference unit
- 26 :: outputting unit

## Claims

1. A reinforcement learning program that causes a computer to execute a process comprising:
executing reinforcement learning of a machine learning model (21a), using a first three-dimensional (3D) molecular structure of a first compound to be adapted to a first object as a state, assignment of a type of an additional atom, a binding position of the additional atom to the first 3D molecular structure, and a 3D position of the additional atom, to the first compound in the state as an action, and an index to evaluate a second 3D molecular structure obtained by binding the additional atom to the first 3D molecular structure in the action as a reward.

2. The reinforcement learning program according to claim 1, wherein
the index comprises a first index related to affinity of biding between a 3D structure of the first object and the second 3D molecular structure.

3. The reinforcement learning program according to claim 2, wherein
the first index comprises an energy difference between a total energy and an entire energy, the total energy being a sum of an energy of the 3D structure of the first object and an energy of the second 3D molecular structure, the entire energy being an energy when a position of binding between the 3D structure of the first object and the second 3D molecular structure is optimum.

4. The reinforcement learning program according to any one of claims 1-3, wherein
the index comprises at least one of a second index and a third index, the second index relating to a likelihood that the second 3D molecular structure is medicine, the third index relating to a difficulty in binding between a 3D structure of the first object and the second 3D molecular structure.

5. The reinforcement learning program according to any one of claims 1-4, wherein
the machine learning model (21a) is a trained machine learning model after being trained so as to output a 3D molecular structure of a second compound to be adapted to each of a plurality of second objects to be input, including the first object, in response to input of the second object into the machine learning model (21a).

6. The reinforcement learning program according to any one of claims 1-5, wherein the process further comprises
outputting a 3D molecular structure of a third compound to be adapted to the first object or a third object, the 3D molecular structure being obtained by inputting the first object or the third object into the machine learning model (21a) after being trained.

7. A computer-implemented method for reinforcement learning comprising:
executing reinforcement learning of a machine learning model (21a), using a first three-dimensional (3D) molecular structure of a first compound to be adapted to a first object as a state, assignment of a type of an additional atom, a binding position of the additional atom to the first 3D molecular structure, and a 3D position of the additional atom, to the first compound in the state as an action, and an index to evaluate a second 3D molecular structure obtained by binding the additional atom to the first 3D molecular structure in the action as a reward.

8. The computer-implemented method according to claim 7, wherein
the index comprises a first index related to affinity of biding between a 3D structure of the first object and the second 3D molecular structure.

9. The computer-implemented method according to claim 8, wherein
the first index comprises an energy difference between a total energy and an entire energy, the total energy being a sum of an energy of the 3D structure of the first object and an energy of the second 3D molecular structure, the entire energy being an energy when a position of binding between the 3D structure of the first object and the second 3D molecular structure is arranged optimally.

10. The computer-implemented method according to any one of claims 7-9, wherein
the index comprises at least one of a second index and a third index, the second index relating to a likelihood that the second 3D molecular structure is medicine, the third index relating to a difficulty in binding between a 3D structure of the first object and the second 3D molecular structure.

11. The computer-implemented method according to any one of claims 7-10, wherein
the machine learning model (21a) is a trained machine learning model after being trained so as to output a 3D molecular structure of a second compound to be adapted to each of a plurality of second objects to be input, including the first object, in response to input of the second object into the machine learning model (21a).

12. The computer-implemented method according to any one of claims 7-11, the computer-implemented method further comprising
outputting a 3D molecular structure of a third compound to be adapted to the first object or a third object, the 3D molecular structure being obtained by inputting the first object or the third object into the machine learning model (21a) after being trained.

13. An information processing apparatus comprising a controller (27), the controller being configured to execute a process comprising
executing reinforcement learning of a machine learning model (21a), using a first three-dimensional (3D) molecular structure of a first compound to be adapted to a first object as a state, assignment of a type of an additional atom, a binding position of the additional atom to the first 3D molecular structure, and a 3D position of the additional atom, to the first compound in the state as an action, and an index to evaluate a second 3D molecular structure obtained by binding the additional atom to the first 3D molecular structure in the action as a reward.

14. The information processing apparatus according to claim 13, wherein
the index comprises a first index related to affinity of biding between a 3D structure of the first object and the second 3D molecular structure.

15. The information processing apparatus according to claim 13 or 14, wherein
the controller (27) outputs a 3D molecular structure of a third compound to be adapted to the first object or a third object, the 3D molecular structure being obtained by inputting the first object or the third object into the machine learning model (21a) after being trained.
